(19)

**Europäisches Patentamt**
**European Patent Office**
**Office européen des brevets**

(11)  **EP 4 738 378 A2**

(12)  **EUROPEAN PATENT APPLICATION**

(43) Date of publication:
06.05.2026   Bulletin 2026/19

(51) International Patent Classification (IPC):
*G16H 20/40* (2018.01)

(21) Application number: 26167317.2

(22) Date of filing: **30.06.2022**

(52) Cooperative Patent Classification (CPC):
**A61B 3/0025; G16H 20/40; G16H 50/20;
G16H 50/30; G16H 50/70;** A61F 2240/002

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   06.07.2021   US 202163218638 P

(62) Document number(s) of the earlier application(s) in
accordance with Art. 76 EPC:
**22743905.6 / 4 366 597**

(71) Applicant: **Alcon Inc.**
**1701 Fribourg (CH)**

(72) Inventors:
• **CAMPIN, John Alfred**
**Fort Worth, Texas 76134 (US)**
• **GRUENDIG, Martin**
**14513 Teltow (DE)**

• **HERNANDEZ, Victor Manuel**
**Fort Worth, Texas 76134 (US)**
• **PETTIT, George Hunter**
**Fort Worth, Texas 76134 (US)**
• **ZIELKE, Mark Andrew**
**Lake Forest, CA 92630 (US)**
• **NEKRASSOV, Daniil**
**14513 Teltow (DE)**

(74) Representative: **Murgitroyd & Company**
**165-169 Scotland Street**
**Glasgow G5 8PL (GB)**

Remarks:
This application was filed on 24.03.2026 as a
divisional application to the application mentioned
under INID code 62.

(54)   **SYSTEM AND METHOD FOR SELECTION OF A PREFERRED INTRAOCULAR LENS**

(57)   A system for selecting a preferred intraocular lens for implantation into an eye includes a controller having a processor and a tangible, non-transitory memory. The controller is configured to obtain diagnostic data of the eye, and obtain historical data composed of historical sets of patient data. The controller is configured to analyze individual risk factors based on the diagnostic data and obtain a weighted combination of the individual risk factors. A respective satisfaction metric for the plurality of intraocular lenses is generated based on the historical data. A preferred intraocular lens may be selected based in part on the respective satisfaction metric and the weighted combination. A visual simulation for each of the plurality of intraocular lenses may be performed, based in part on the diagnostic data. The visual simulation may incorporate an impact of the tear film data.

EP 4 738 378 A2

## Description

INTRODUCTION

The disclosure relates generally to a system for selecting a preferred intraocular lens for implantation in an eye. The human lens is generally transparent such that light may travel through it with ease. However, many factors may cause areas in the lens to become cloudy and dense, and thus negatively impact vision quality. The situation may be remedied via a cataract procedure, whereby an artificial lens is selected for implantation into a patient's eye. Indeed, cataract surgery is commonly performed all around the world. Traditionally, cataract patients who underwent surgery received an artificial lens designed to enhance distance vision only. Many patients suffered from varying levels of post-surgical presbyopia, which required the use of reading glasses or bifocals. Today different types of advanced technology intraocular lenses, such as multifocal intraocular lenses, are available for correcting a number of vision variances. Current screening methods for advanced technology intraocular lenses require in-depth expertise of the surgeon and are time consuming to carry out. As a result, surgeons may be hesitant to prescribe advanced technology intraocular lenses.

SUMMARY

Disclosed herein is a system for selecting a preferred intraocular lens for implantation into an eye. The system includes a controller having a processor and a tangible, non-transitory memory on which instructions are recorded. The controller is configured to obtain diagnostic data of the eye. The controller is configured to obtain historical data composed of historical sets of patient data. The controller is configured to analyze individual risk factors based on the diagnostic data and obtain a weighted combination of the individual risk factors. A respective satisfaction metric for the plurality of intraocular lenses is generated based on the historical data.

The controller is configured to select the preferred intraocular lens based in part on the respective satisfaction metric and the weighted combination. A visual simulation for each of the plurality of intraocular lenses may be performed, based in part on the diagnostic data. The respective satisfaction metric may be based in part on the visual simulation. The diagnostic data may include tear film data. The visual simulation may incorporate an impact of the tear film data, including detecting a respective location where the tear film data exhibits at least one of a change in a signal-to-noise ratio and a relatively lower signal-to-noise ratio than that of surrounding locations, and identifying the respective location as a respective irregularity of a tear film in the eye. Incorporating the impact of the tear film data may include identifying a respective location where the tear film data exhibits at least one of missing information and a varying point distribution, and identifying the respective location as a respective irregularity of a tear film.

The diagnostic data may include corneal data represented as at least one of a binary result or as a numerical scale of irregular corneal aberrations, the eye being scanned to generate the diagnostic data. The binary result may be either a presence of a threshold level of corneal aberrations or an absence of the threshold level of corneal aberrations. The diagnostic data may include macular data represented as at least one of a binary result or as a numerical scale of macular degeneration the eye being scanned to generate the diagnostic data. The binary result may be either a presence of a threshold level of degeneration or an absence of the threshold level of degeneration. The diagnostic data may include a respective location, orientation, and size of a pupil of the eye in a three-dimensional coordinate system, the pupil being under photopic conditions, and the respective location and respective profile of an anterior corneal surface and a posterior corneal surface of the eye.

The diagnostic data may include lens capsule stability data represented by one or more wobble parameters. Obtaining the lens capsule stability data may include acquiring a plurality of images of the eye while presenting different accommodative demands to the eye and generating a motion trace of a lens capsule of the eye using the plurality of images. Obtaining the lens capsule stability data may further include extracting normalized lens oscillation traces based on the motion trace, model-fitting a curve to the normalized lens oscillation traces and obtaining the one or more wobble parameters as a maximum amplitude and/or a time constant of the curve.

Obtaining the lens capsule stability data may include directing electromagnetic energy in a predetermined spectrum onto the eye concurrently with induced eye saccades, via an energy source, and acquiring a plurality of images of the eye indicative of the induced eye saccades, via a camera. Obtaining the lens capsule stability data may further include generating a motion trace of a lens capsule using the plurality of images and extracting normalized lens oscillation traces based on the motion trace, model-fitting a curve to the normalized lens oscillation traces and obtaining the one or more wobble parameters based on the curve.

The diagnostic data may include an angle kappa factor. The diagnostic data may include questionnaire data for the patient with at least one personality trait, the at least one personality trait being represented as at least one of a numerical scale of agreeability or as a binary result, the binary result being either predominantly agreeable or predominantly non-agreeable.

Determining the respective satisfaction metric may include selectively executing at least one machine learning model trained with the respective historical sets. The respective historical sets include pre-operative objective data, pre-operative personality data, intra-operative data, post-operative objective data, and subjective outcome data. The subjective outcome data in the respective historical sets may include a numerical satisfaction scale. The controller is configured to quantify a correlation of the post-operative objective data to the subjective outcome score in the respective historical sets and identify the post-operative objective data most strongly correlating with the subjective outcome score.

Disclosed herein is a method of selecting a preferred intraocular lens for implantation in an eye, with a system having a controller with a processor and a tangible, non-transitory memory on which instructions are recorded. The method includes obtaining diagnostic data for the eye and analyzing individual risk factors based on the diagnostic data, via the controller. Historical data composed of respective historical sets of patient data are obtained. The method includes obtaining a weighted combination of the individual risk factors based in part on the historical data, via the controller. A respective satisfaction metric is generated for the plurality of intraocular lenses based on the historical data, via the controller.

The above features and advantages and other features and advantages of the present disclosure are readily apparent from the following detailed description of the best modes for carrying out the disclosure when taken in connection with the accompanying drawings.

BRIEF DESCRIPTION OF THE DRAWINGS

FIG. 1 is a schematic illustration of a system for selecting a preferred intraocular lens for implantation into an eye;
FIG. 2 is a schematic flowchart of a method for selecting a preferred intraocular lens;
FIG. 3 is a schematic diagram illustrating one or more irregularities in a tear film of the eye of FIG. 1;
FIG. 4 is a schematic diagram of a set-up for obtaining lens capsule stability data for the system of FIG. 1; and
FIG. 5 is a schematic example of a graph of lens capsule stability data obtained by the set-up of FIG. 4.

Representative embodiments of this disclosure are shown by way of nonlimiting example in the drawings and are described in additional detail below. It should be understood, however, that the novel aspects of this disclosure are not limited to the particular forms illustrated in the above-enumerated drawings. Rather, the disclosure is to cover modifications, equivalents, combinations, sub-combinations, permutations, groupings, and alternatives falling within the scope of this disclosure as encompassed, for instance, by the appended claims.

DETAILED DESCRIPTION

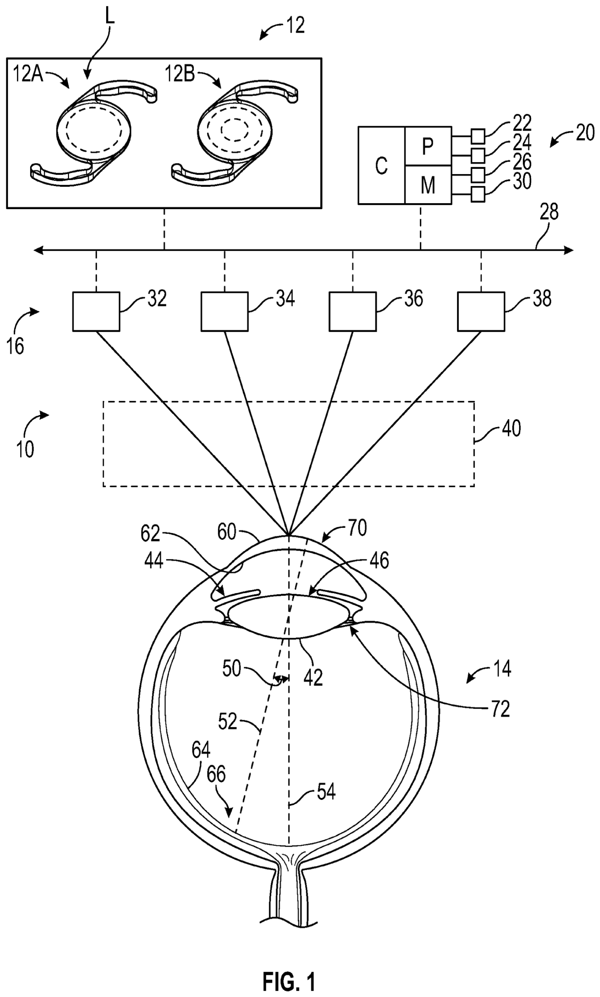

Referring to the drawings, wherein like reference numbers refer to like components, FIG. 1 schematically illustrates a system 10 for selecting a preferred intraocular lens L for implantation in an eye 14 of a patient. The preferred intraocular lens L is selected from a plurality of intraocular lenses 12, which may be advanced technology lenses. The plurality of intraocular lenses 12 may include a first IOL 12A and a second IOL 12B, which may be mono-focal or multifocal lenses of varying powers. In some embodiments, the first IOL 12A is configured to provide better vision in a first distance range and the second IOL 12B is configured to provide better vision in a second distance range. Alternatively, first IOL 12A may be an accommodating lens with a fluid-filled internal cavity, the fluid being movable in order to vary a thickness (and power) of the first IOL 12A. It is to be understood that the plurality of intraocular lenses 12 may take many different forms and include multiple and/or alternate components.

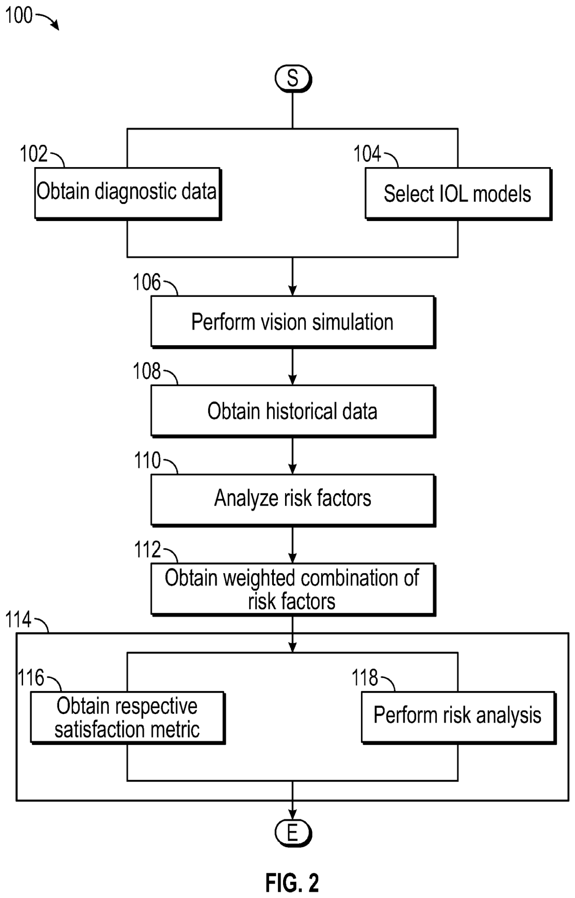

Referring to FIG. 1, the system 10 includes a controller C having at least one processor P and at least one memory M (or non-transitory, tangible computer readable storage medium) on which instructions are recorded for executing a method 100 for selecting the preferred intraocular lens L. Method 100 is shown in and described below with reference to FIG. 2.

Referring to FIG. 1, the controller C is configured to obtain diagnostic data of the eye 14, via one or more imaging devices 16. As described below, the system 10 receives a diverse range of diagnostic data relevant for the prediction of patient satisfaction. The diagnostic data may function as indicators independently, or in combination with each other. These inputs may be generated with a single diagnostic tool, several diagnostic tools, or entered manually. Examples of diagnostic data include the size, shape, and position of the pupil, the angle kappa, irregular corneal aberrations, tear film dynamics, macular pathology, patient questionnaire and lens stability data (lens wobble or phacodonesis). The controller C is configured to obtain a simulated visual performance of the eye 14, based on the diagnostic data.

The system 10 individually analyzes each of the diagnostic inputs and simulated metrics for their potential to impact patient satisfaction. Many patients would benefit from the extended range of vision that comes with advanced technology intraocular lenses but do not have access to this technology due to the time-consuming selection process. The system 10 provides patient satisfaction metrics to the clinician and highlights primary risk drivers, reducing the time and burden involved. The system 10 may learn and improve over time, and the data may be shared across sites.

The controller C may include a plurality of modules 20 selectively executable by the controller C, including a diagnostic module 22, a simulation module 24, and a prediction module 26. The plurality of modules 20 may be embedded in the controller C. The plurality of modules 20 may be a part of a remote server or cloud unit (not shown) accessible to the controller C via a network 28. The diagnostic module 22 is adapted to store the diagnostic data, which may be in the form of an eye model. The simulation module 24 is adapted to perform a visual simulation based on the diagnostic data. The prediction module 26 is adapted to generate a respective satisfaction metric for the plurality of intraocular lenses 12 based on the visual simulation and historical data. Referring to FIG. 1, the system 10 may include a lens selection module 30 that receives the output of the prediction module 26 for a set of intraocular lenses for investigation, i.e., the plurality of intraocular lenses 12.

The various components of the system 10 may be configured to communicate via the network 28, shown in FIG. 1. The network 28 may be a bidirectional bus implemented in various ways, such as for example, a serial communication bus in the form of a local area network. The local area network may include, but is not limited to, a Controller Area Network (CAN), a Controller Area Network with Flexible Data Rate (CAN-FD), Ethernet, WIFI, Bluetooth™ and other forms of data connection. Other types of connections may be employed. The system 10 may include a user interface (not shown) operable by a user, which may include a touchscreen or other input device. The controller C may be configured to process signals to and from the user interface and a display (not shown).

Referring now to FIG. 2, a flowchart of the method 100 is shown. Method 100 may be embodied as computer-readable code or instructions stored on and partially executable by the controller C of FIG. 1. The method 100 need not be applied in the specific order recited herein and may be dynamically executed. Furthermore, it is to be understood that some steps may be eliminated. As used herein, the terms 'dynamic' and 'dynamically' describe steps or processes that are executed in real-time and are characterized by monitoring or otherwise determining states of parameters and regularly or periodically updating the states of the parameters during execution of a routine or between iterations of execution of the routine.

Per block 102 of FIG. 2, the controller C is configured to obtain diagnostic data of the eye 14, via the imaging devices 16. The imaging devices 16 may include an optical coherence tomography (OCT) device 32, an aberrometer 34, a reflection topographer 36 and a photo-sensing device, such as a camera 38. The imaging devices 16 may include an ultrasound or magnetic resonance imaging machine (not shown) or other imaging device available to those skilled in the art. The diagnostic data may be derived from a single image or from multiple images. The measurements from the different imaging devices 16 are aligned so that the measurement data corresponds to the same location in the eye 14. An optical unit 40 may be positioned between the eye 14 and the imaging devices 16 in order to direct light from the imaging devices 16 towards the eye 14. The optical unit 40 may include optical elements available to those skilled in the art, such as for example, a lens, prism, mirror, diffractive optical element, holographic optical element, and spatial light modulator.

Referring to FIG. 1, the diagnostic data includes a position, orientation (e.g., tilt) and size of the crystalline lens 42 and iris 44, respectively. The diagnostic data includes the position, orientation and size of the pupil 46 under photopic conditions. Photopic conditions refer to vision under well-lit conditions, which functions primarily due to cone cells in the eye. In some embodiments, photopic conditions may be defined to cover adaptation levels of 3 candelas per square meter $(cd/m^2)$ and higher.

Referring to FIG. 1, the diagnostic data may include a thickness of the lens 42, anterior chamber depth and the refractive indices of different portions of the eye 14. The diagnostic data may include an angle kappa 50, defined as the angle between a visual axis 52 (connecting the center of the pupil 46 with the fovea) and a pupillary axis 54 (perpendicularly passing through the entrance pupil and the center of curvature of the cornea). A relatively large value of the angle kappa 50 may contribute to inadvertent decentration of the implanted intraocular lens and other potential issues.

Referring to FIG. 1, the diagnostic data may include corneal data, such as corneal thickness, and the shape and location of the anterior corneal surface 60 and the posterior corneal surface 62. The imaging devices 16 may measure various surfaces of the eye 14 by analyzing light reflected from the eye 14 (e.g., from an illumination source). For example, the OCT device 32 detects reflections from points of the anterior corneal surface 60 and converts their optical path lengths to distances to yield a point distribution of the anterior corneal surface 60. The OCT device 32 may output OCT measurement data in any suitable manner, e.g., as distances, a point distribution, a topology, an ocular model, and/or a map. The OCT device 32 may use time domain, frequency domain, or other suitable spectral encoding, and may use single point, parallel, or other type of scanning pattern.

In another example, the aberrometer 34 uses wavefront technology to determine the aberrations of eye 14. As a wavefront of light travels through eye 14 and is reflected back through eye 14, aberrations of eye 14 distort the shape of the wavefront from an ideal shape. The aberrometer 34 generates measurement data that describe the deviations of the measured wavefront from the ideal wavefront. For example, the reflection topographer 36 measures the shape of the anterior corneal surface 60 of eye 14 by detecting how the anterior corneal surface 60 reflects a projected illumination pattern (e.g., concentric rings or grid of dots). If the anterior corneal surface 60 is an ideal sphere, the reflected pattern matches the projected pattern. If the anterior corneal surface 60 has aberrations, areas where the reflected portions of the pattern are closer together may indicate steeper corneal curvature, and areas where the portions are farther part may indicate flatter areas. Application 63/126441 (filed 16 December 2020) describes multidetector analyses of the tear film of an eye and is incorporated by reference in its entirety.

The corneal data may be represented as at least one of a binary result or as a numerical scale of irregular corneal aberrations. The binary result may be either a presence of a threshold level of corneal aberrations (e.g., a percentage of the corneal surface) or an absence of the threshold level of corneal aberrations. The controller C may be selectively executable to approximate or parametrize surfaces in the eye 14 based on the diagnostic data and algorithms available to those skilled in the art.

Referring to FIG. 1, the diagnostic data may include an approximation of the surface of the retina 64 from an axial length of the eye 14 (assuming a near spherical shape of the ocular globe). In some embodiments, the diagnostic data includes macular data, represented as at least one of a binary result or as a numerical scale of macular degeneration. The macula 66 is a portion of the retina 64 with a high concentration of photoreceptor cells. It is responsible in part for central vision, color vision and fine details. Diseases of the macula, such as age-related macular degeneration, interfere with the sharp, central vision needed for activities such as reading. The binary result may be either a presence of a threshold level of macular degeneration or an absence of the threshold level of macular degeneration.

In some embodiments, the diagnostic data includes tear film data, which may indicate irregularities of the tear film 70. Referring to FIG. 1, the tear film 70 on the anterior corneal surface 60 protects and lubricates the eye 14. The tear film 70 washes away foreign particles and reduces the risk of eye infection. The irregularity may be a deviation from the normal tear film, e.g., an area where the tear film 70 is absent, abnormally thin or has a different chemical composition. The irregularity may be an instability in the tear film 70, e.g., an area where the tear film 70 changes rapidly.

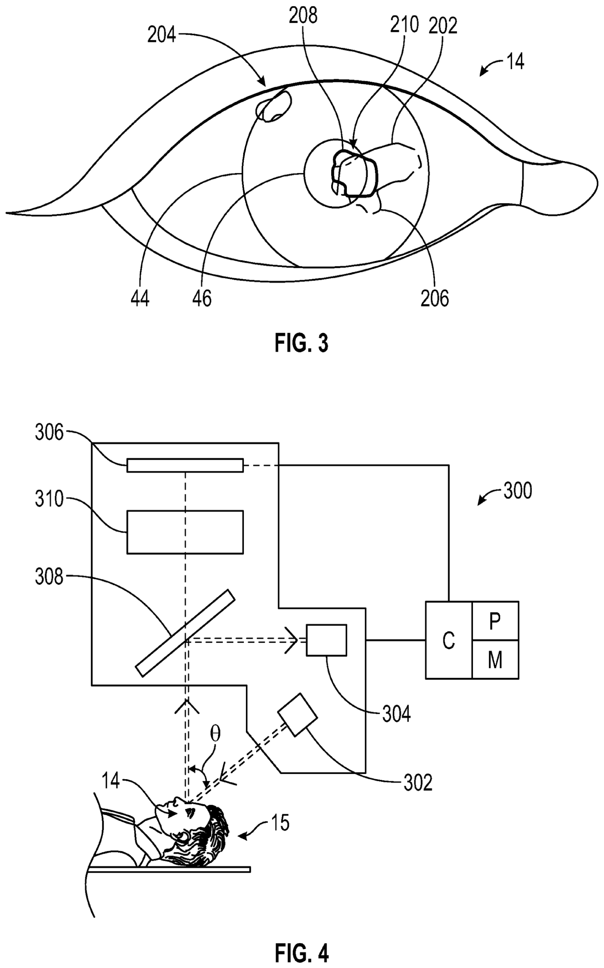

FIG. 3 is a schematic diagram of an eye 14 (having iris 44 and pupil 46) with one or more irregularities in the tear film. FIG. 3 illustrates a first irregularity 202 detected by a first device (e.g., OCT device 32), a second irregularity 204 detected by a second device (e.g., topographer 36) and a third irregularity 206 detected by a third device (e.g., aberrometer 34). In some embodiments, the controller C is configured to create a composite irregularity 208 based on the overlap between two irregularities detected by two different devices. For example, composite irregularity 208 is generated based in the overlapping region between first irregularity 202 and the third irregularity 206.

The controller C may be adapted to assess an impact of the tear film data in a number of ways, such as for example, detecting a respective location 210 where the tear film data exhibits a change in a signal-to-noise ratio, where the tear film data exhibits a relatively lower signal-to-noise ratio than that of surrounding locations and identifying the respective location 210 as an irregularity of the tear film. The signal-to-noise ratio of data from a location is the ratio of the measured signal to the overall measured noise at the location. A respective location 210 with a lower or decreased signal-to-noise ratio may indicate an issue with the tear film 70. In some embodiments, the controller C assesses an impact of the tear film data according to the presence or absence of data, with absent data indicating an irregularity at the location, and/or whether a respective point distribution of the tear film data is changing.

In some embodiments, the diagnostic data includes lens capsule stability data. The outer periphery of the lens capsule is attached to a ring of elastic fibers, generally referred to as Zinn's membrane or zonules. Ciliary muscles 72 (see FIG. 1) within the eye 14 contract or relax to collectively act on the zonules during accommodation, which has the effect of changing the shape of the lens capsule. When the zonules are excessively resilient, the lens and the capsular bag may become less securely attached to the ciliary muscles 72 and the patient 15 may be at an increased risk for certain complications during an implantation. A surgeon may attempt to mitigate surgical risk by employing a capsular support device to stabilize the capsular bag, by performing a laser-based capsular rhexis procedure, or by taking other precautionary measures.

FIG. 4 is a schematic diagram of a set-up 300 for obtaining lens capsule stability data in the eye 14 of a patient 15. Referring to FIG. 4, an energy source 302 is adapted to direct electromagnetic energy (e.g., IR or visible light, ultrasonic energy) onto a pupil of the eye 14. The electromagnetic energy may be directed at a predetermined intensity level sufficient for inducing characteristic Purkinje reflexes or other ocular reflexes in the pupil of the eye 14.

The lens capsule stability data may be represented as a numerical scale of ciliary muscle activity of the eye 14 and/or as one or more wobble parameters. Obtaining the ciliary muscle activity includes presenting different accommodative demands to the eye 14, while acquiring a plurality of images, via a camera 304 (e.g., a high-speed camera). Application 63/129386 (filed 22 Dec 2020) describes an assessment of human lens capsule stability and is incorporated by reference in its entirety. Referring to FIG. 4, in some embodiments, the eye 14 is directed towards a visual target 306 arranged along the patient's line-of-sight, e.g., via a dynamic gaze-guiding cue transmitted by the controller C. The gaze-guiding cue induces predetermined and controlled eye movements, referred to herein as eye saccades. A saccade is a rapid eye movement that shifts the center of gaze from one part of the visual field to another. Saccades are generally used for orienting gaze towards an object of interest, and may be horizontal, vertical or oblique.

Referring to FIG. 4, a hot mirror 308 may be arranged at a predetermined angle θ with respect to the camera 304 and configured to direct reflected light from the eye 14 toward the camera 304. The plurality of images of the eye 14 are indicative of the eye saccades. The set-up 300 may include one or more optical devices 310 for directing the light. The stability of the lens capsule may be assessed by fitting the movement of a fourth Purkinje image (reflection from the posterior surface of the crystalline lens 42) relative to a first Purkinje image (reflection from the anterior corneal surface 60). For example, the relative movement may be fitted to the following damped harmonic model:

$$\frac{\partial^2 \varphi}{\partial t^2} + 2\beta\omega\frac{\partial \varphi}{\partial t} + \omega\varphi = 0$$

Here φ is the relative position of the fourth Purkinje image with respect to the first Purkinje image; t represents time; β is the damping ratio and ω is the undamped angular frequency of the movement.

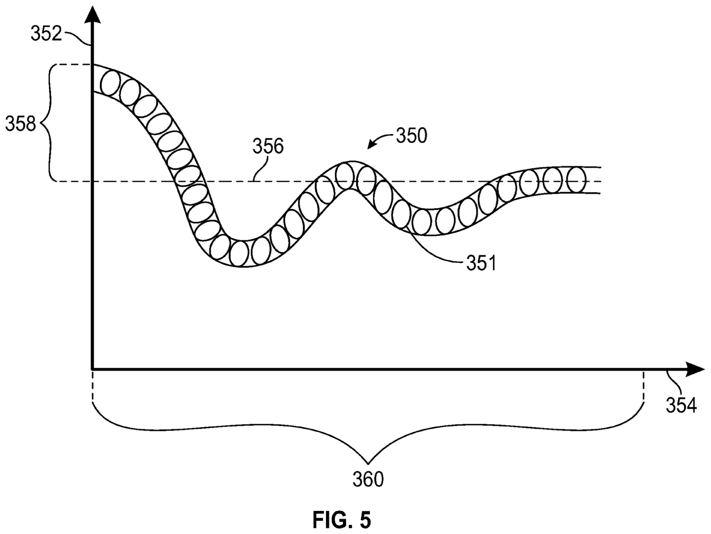

The controller C is configured to calculate motion curves of the lens capsule of the eye 14 using the plurality of images from the camera 304. The controller C is adapted to extract normalized lens oscillation traces based on the motion curve and model-fit a curve to the normalized lens oscillation traces. Referring to FIG. 5, an example fitted curve 350 derived from an example set of lens capsule stability data (with normalized lens oscillation traces 351) is shown. The vertical axis 352 in FIG. 5 indicates the relative position of the fourth Purkinje image with respect to the first Purkinje image, with line 356 indicating a zero difference in position. The horizontal axis 354 in FIG. 5 shows time. In one embodiment, a lumped mass model is used to perform the model-fitting of the lens oscillation traces. The fitted curve 350 may be employed to obtain the wobble parameters. For example, the wobble parameters may be represented by a maximum amplitude 358 (where wobbling is at a maximum), damping ratio and a time constant 360 (where the amplitude has settled down to zero) of the fitted curve 350.

The diagnostic data may include questionnaire data for the patient 15, with the questionnaire data assessing or reflecting upon at least one personality trait (which may be self-reported). The personality trait may be represented as at least one of a numerical scale of agreeability (i.e., how agreeable the patient 15 is). The personality trait may be represented as a binary result, for example, as either predominantly agreeable or predominantly non-agreeable.

Per block 104 of FIG. 2, the controller C is configured to select a plurality of intraocular lenses 12 (see FIG. 1) to be investigated for implantation into the eye 14 and obtain a respective IOL model for each of the plurality of intraocular lenses 12. In some embodiments, the IOL model is a diffractive model, i.e., based on modelling diffractive surfaces.

The method 100 proceeds from blocks 102 and 104 to block 106. Per block 106, the controller C is programmed to perform visual simulation for each of the plurality of intraocular lenses 12, based on the diagnostic data from block 102 and the respective IOL models from block 104. This may be done via the simulation module 24 embedded in or otherwise in communication with the controller C.

The output of the visual simulation may include focus curves, simulated visual acuity, and contrast sensitivity. The output of the visual simulation may include a wavefront distribution, a modulation transfer function (MTF) and a point spread function (PSF). The modulation transfer function is formally defined as the magnitude (absolute value) of the complex optical transfer function, which specifies how different spatial frequencies are handled by an optical system.

The simulation module 24 may be configured to employ ray tracing to assess the focusing properties of the plurality of intraocular lenses 12. In other words, the propagation of light through the eye 14 may be traced through reflection and refraction using Snell's law, which describes the refraction of a ray at a surface separating two media with different refractive indices. The spatial distribution of a bundle of rays traced or propagated to a spot on the retina 64 may be used to derive a respective visual acuity score at a specific distance. The refractive indices applicable to a multitude of wavelengths may be employed. This helps to account for chromatic dispersion effects, for example, between a diagnostic measurement wavelength and different wavelengths of importance to human vision, or between multiple visible wavelengths to assess the impact of chromatic aberration on retinal image quality and other factors. The visual simulation incorporates an impact of tear film dynamics. In other words, the propagation of light through the eye 14 is affected by the irregularities (e.g., composite irregularity 208 shown in FIG. 3) obtained in block 102.

The simulation module 24 is adapted to estimate post-operative anatomic parameters of the eye 14, such as predicted lens tilt and a predicted lens decentration. Post-operatively, a pupil 46 may be decentered or tilted with respect to the visual axis 52. Post-operatively, the iris 44 may assume a relatively planar geometry, while preoperatively, the iris 44 may be bulging and shifted anteriorly due to the relatively bulkier shape of the crystalline lens 42. The simulation module 24 may employ intraocular lens power calculation formula available to those skilled in the art. Examples of such formulas include the SRK/T formula, the Holladay formula, the Hoffer Q formula, the Olsen formula and the Haigis formula.

The method 100 proceeds from block 106 to block 108. Per block 108 of FIG. 2, the controller C is configured to obtain historical data composed of historical sets of patient data. The historical data includes pre-operative objective data, pre-operative personality data, intra-operative data, post-operative objective data, and subjective outcome data of the same person. The pre-operative objective data and post-operative objective data may include anatomic eye measurements (e.g.,

eye length, corneal topography and thickness, lens position and thickness, etc.), refractive eye measurements (e.g., classical refraction, wavefront aberrometry), visual function measurements (e.g., photopic/mesopic visual acuity, contrast sensitivity, near vision, etc.) and physiologic eye measurements (e.g., intraocular pressure, tear film health, etc.) The pre-operative personality data may include a visual needs assessment or lifestyle demands (dominant activities, e.g., needlepoint versus fishing) and a personality trait. In one example, the Big Five Factor model of personality type, sometimes known as McCrae and Costa, may be employed. The Big Five Factor model posits that the traits of openness, agreeableness conscientiousness, extraversion and neuroticism (or emotional stability) form the basis of people's personalities (see McCrae, R., Costa, P., Personality in Adulthood: A Five-Factor Theory Perspective, Guilford Press, New York City (2003).

Examples of intra-operative data include, but are not limited to, the type of refractive surgery procedure performed, the model of the implanted intraocular lens and its prescription. The intra-operative data may include intra-operative aberrometry measurements. The intra-operative data may further include the surgical machine settings and parameters of the procedure, such as procedure time, the temperature of the operating room, the total phaco power consumed to emulsify the original lens, the time duration that the phaco energy was applied, and the effective phaco time (as a product of phaco time multiplied by an average phaco power). The intra-operative data may further include: the type of delivery device used to implant the intraocular lens, the presence or absence of any occlusion breaks, the quantity and degree of the occlusion breaks, and whether or not assistive devices (such as capsular hooks) were employed. The intra-operative data may further include an intra-operative grade of nuclear hardness of the original lens, which may be graded according to a lens opacity classification.

The subjective outcome data in the respective historical sets may include one or more numerical satisfaction scale that reflects satisfaction with the post-operative visual outcome. The patient's satisfaction with their surgical outcome may be captured at one or more specific time periods (e.g., at 1 month and at 3 months post-surgery). In one example, a single overall satisfaction is employed, based on the following question: "on a scale of 1-5 (with 5 being best), how happy are you with your vision now?" In another example, separate satisfaction scales may be employed for near vision, far vision, night/dim light vision, "outdoor sports vision" (e.g. playing golf) and overall satisfaction. Other examples of historical data include best-corrected near visual acuity, best-corrected far visual acuity and lens capsule stability evaluation.

Also, per block 108, the method 100 includes training one or more machine learning models based on the historical data. The trained machine learning models are employed to obtain a weighted combination of individual risk factors (obtained in block 110), as will be described below with respect to block 112. The machine learning models may include a neural network algorithm, a multi-layer perceptron network, a support vector regression model or any other model available to those skilled in the art. For example, neural networks recognize patterns from real-world data (e.g., images, sound, text, time series and others) that is translated or converted into numerical form and embedded in vectors or matrices. The neural network may employ deep learning maps to match an input vector $x$ to an output vector $y$. The training process enables the neural network to correlate the appropriate activation function $f(x)$ for transforming the input vector $x$ to the output vector $y$. Once the machine learning model is trained with the historical data, estimated values of the output vector $y$ may be computed with given new values of the input vector $x$. It is understood that other types of machine learning models may be employed.

The method 100 proceeds from block 108 to block 110. Per block 110 of FIG. 2, the controller C is configured to analyze individual risk factors for the eye 14 based on the diagnostic data (block 102), historical data (block 108) and the visual simulation (block 106). The individual risk factors include potential issues arising from the presence of irregular corneal aberrations, tear film dynamics, presence of macular degree, size of the angle kappa 50 and the size of wobble parameters of the eye 14 (from lens capsule stability data). The controller C may be configured to quantify a correlation of the respective post-operative objective data to the respective subjective outcome score in the historical data and identify the respective post-operative objective data most strongly correlating with the respective subjective outcome score.

Next, per block 112 of FIG. 2, the controller C is configured to obtain a weighted combination of the individual risk factors based on the historical data (block 108), via the one or more machine learning models trained with the historical data. The historical data may be stratified based on demographic data, patients with similar-sized dimensions of eyes or other health status factors. The historical data may be updated periodically. The system 10 may be configured to be adaptive, with the machine learning models being re-trained periodically based on the updated data.

The method 100 proceeds to block 114 from block 112, where the controller C is configured to generate a number of outputs, shown in sub-blocks 116 and 118. This information allows the clinician to select the best model and/or power to optimize visual performance and minimize risk. The system 10 individually analyzes each of the diagnostic inputs (from the diagnostic data) and simulated metrics for their potential to impact patient satisfaction. The application will also provide one or more metrics to the surgeon, which represent patient satisfaction based on the diagnostic data. An example of this would be the percentile of patients with similar metrics that are satisfied with their advanced technology intraocular lens.

Per sub-block 116 of FIG. 2, the controller C is programmed to generate a respective satisfaction metric for the plurality of intraocular lenses 12 based on the diagnostic data (block 102), visual simulation (block 106) and the historical data (block 108). This may be done via the prediction module 26. Different dynamic models may be applied, in combination with the historical data. In some embodiments, the prediction module 26 incorporates a machine learning module (such as a neural network) which is trained using a training dataset composed at least partially of the historical data.

Per sub-block 118 of FIG. 2, the controller C may be programmed to present a risk assessment for the patient 15, based on the weighted combination of the individual risk factors (obtained in block 112) and recommend a preferred intraocular lens L. In one embodiment, the selection is automated via a lens selection module 30 that highlights or groups the plurality of intraocular lenses 12 within a predefined acceptable level of individual risk factors (or meeting a predefined minimum level of the respective satisfaction metric).

In summary, the controller C is configured to obtain diagnostic data of the eye 14 and perform visual simulation for each of a plurality of intraocular lenses 12. The visual simulation incorporates an impact of tear film dynamics and other diagnostic data. The controller C is configured to analyze individual risk factors based on the diagnostic data and the visual performance and generate a respective satisfaction metric. The system 10 provides the clinician with an objective and data-driven approach to select well-suited patients for selection of intraocular lenses, such as advanced technology intraocular lenses.

The controller C of FIG. 1 includes a computer-readable medium (also referred to as a processor-readable medium), including a non-transitory (e.g., tangible) medium that participates in providing data (e.g., instructions) that may be read by a computer (e.g., by a processor of a computer). Such a medium may take many forms, including, but not limited to, non-volatile media and volatile media. Non-volatile media may include, for example, optical or magnetic disks and other persistent memory. Volatile media may include, for example, dynamic random-access memory (DRAM), which may constitute a main memory. Such instructions may be transmitted by one or more transmission media, including coaxial cables, copper wire and fiber optics, including the wires that comprise a system bus coupled to a processor of a computer. Some forms of computer-readable media include, for example, a floppy disk, a flexible disk, hard disk, magnetic tape, other magnetic medium, a CD-ROM, DVD, other optical medium, punch cards, paper tape, other physical medium with patterns of holes, a RAM, a PROM, an EPROM, a FLASH-EEPROM, other memory chip or cartridge, or other medium from which a computer can read.

Look-up tables, databases, data repositories or other data stores described herein may include various kinds of mechanisms for storing, accessing, and retrieving various kinds of data, including a hierarchical database, a plurality of files in a file system, an application database in a proprietary format, a relational database management system (RDBMS), etc. Each such data store may be included within a computing device employing a computer operating system such as one of those mentioned above and may be accessed via a network in one or more of a variety of manners. A file system may be accessible from a computer operating system and may include files stored in various formats. An RDBMS may employ the Structured Query Language (SQL) in addition to a language for creating, storing, editing, and executing stored procedures, such as the PL/SQL language mentioned above.

The flowcharts presented herein illustrate an architecture, functionality, and operation of possible implementations of systems, methods, and computer program products according to various embodiments of the present disclosure. In this regard, each block in the flowchart or block diagrams may represent a module, segment, or portion of code, which comprises one or more executable instructions for implementing the specified logical function(s). It will also be noted that each block of the block diagrams and/or flowchart illustrations, and combinations of blocks in the block diagrams and/or flowchart illustrations, may be implemented by specific purpose hardware-based devices that perform the specified functions or acts, or combinations of specific purpose hardware and computer instructions. These computer program instructions may also be stored in a computer-readable medium that can direct a controller or other programmable data processing apparatus to function in a particular manner, such that the instructions stored in the computer-readable medium produce an article of manufacture including instructions to implement the function/act specified in the flowchart and/or block diagram blocks.

The numerical values of parameters (e.g., of quantities or conditions) in this specification, including the appended claims, are to be understood as being modified in each respective instance by the term "about" whether or not "about" actually appears before the numerical value. "About" indicates that the stated numerical value allows some slight imprecision (with some approach to exactness in the value; about or reasonably close to the value; nearly). If the imprecision provided by "about" is not otherwise understood in the art with this ordinary meaning, then "about" as used herein indicates at least variations that may arise from ordinary methods of measuring and using such parameters. In addition, disclosure of ranges includes disclosure of each value and further divided ranges within the entire range. Each value within a range and the endpoints of a range are hereby disclosed as separate embodiments.

The detailed description and the drawings or FIGS. are supportive and descriptive of the disclosure, but the scope of the disclosure is defined solely by the claims. While some of the best modes and other embodiments for carrying out the claimed disclosure have been described in detail, various alternative designs and embodiments exist for practicing the disclosure defined in the appended claims. Furthermore, the embodiments shown in the drawings or the characteristics of various embodiments mentioned in the present description are not necessarily to be understood as embodiments independent of each other. Rather, it is possible that each of the characteristics described in one of the examples of an embodiment can be combined with one or a plurality of other desired characteristics from other embodiments, resulting in other embodiments not described in words or by reference to the drawings. Accordingly, such other embodiments fall within the framework of the scope of the appended claims. This application is a divisional of EP 22743905.6 (based on PCT/IB2022/056127) the entire contents of which are herein incorporated by reference. Further arrangements of the present specification are set out in the following numbered clauses (based on the clauses of PCT/IB2022/056127 as filed).

Clauses

1. A system for selecting a preferred intraocular lens, from a plurality of intraocular lenses, for implantation into an eye of a patient, the system comprising:

a controller having a processor and a tangible, non-transitory memory on which instructions are recorded, execution of the instructions causing the controller to: obtain diagnostic data of the eye; obtain historical data composed of respective historical sets of patient data;
analyze individual risk factors based on the diagnostic data and obtain a weighted combination of the individual risk factors based in part on the historical data; and generate a respective satisfaction metric for the plurality of intraocular lenses based in part the historical data.

2. The system of clause 1, wherein the controller is configured to: select the preferred intraocular lens based on the respective satisfaction metric and the weighted combination of the individual risk factors.

3. The system of clause 1, wherein the controller is configured to:
perform a visual simulation for each of the plurality of intraocular lenses based in part on the diagnostic data, the respective satisfaction metric being based in part on the visual simulation.

4. The system of clause 3, wherein:
the diagnostic data includes tear film data, the visual simulation incorporating an impact of the tear film data, including: detecting a respective location where the tear film data exhibits at least one of a change in a signal-to-noise ratio and a relatively lower signal-to-noise ratio than that of surrounding locations; and identifying the respective location as a respective irregularity of a tear film in the eye.

5. The system of clause 3, wherein: the diagnostic data includes tear film data, the visual simulation incorporating an impact of the tear film data, including: identifying a respective location where the tear film data exhibits at least one of missing information and a varying point distribution; and identifying the respective location as a respective irregularity of a tear film.

6. The system of clause 1, wherein: the diagnostic data include corneal data represented as at least one of a binary result or as a numerical scale of irregular corneal aberrations, the eye being scanned to generate the diagnostic data; and the binary result is either a presence of a threshold level of corneal aberrations or an absence of the threshold level of corneal aberrations.

7. The system of clause 1, wherein: the diagnostic data include macular data represented as at least one of a binary result or as a numerical scale of macular degeneration the eye being scanned to generate the diagnostic data; and the binary result is either a presence of a threshold level of degeneration or an absence of the threshold level of degeneration.

8. The system of clause 1, wherein the diagnostic data include: a respective location, orientation, and size of a pupil of the eye in a three-dimensional coordinate system, the pupil being under photopic conditions; and the respective location and respective profile of an anterior corneal surface and a posterior corneal surface of the eye.

9. The system of clause 1, wherein: the diagnostic data includes lens capsule stability data represented by one or more wobble parameters, obtaining the lens capsule stability data including: acquiring a plurality of images of the eye while presenting different accommodative demands to the eye; and generating a motion trace of a lens capsule of the eye using the plurality of images.

10. The system of clause 9, wherein obtaining the lens capsule stability data further includes: extracting normalized lens oscillation traces based on the motion trace; model-fitting a curve to the normalized lens oscillation traces; and
obtaining the one or more wobble parameters as a maximum amplitude and/or a time constant of the curve.

11. The system of clause 1, wherein: the diagnostic data includes lens capsule stability data represented by one or more wobble parameters, obtaining the lens capsule stability data including: directing electromagnetic energy in a predetermined spectrum onto the eye concurrently with induced eye saccades, via an energy source; acquiring a plurality of images of the eye indicative of the induced eye saccades, via a camera; generating a motion trace of a lens capsule using the plurality of images and extracting normalized lens oscillation traces based on the motion trace; model-fitting a curve to the normalized lens oscillation traces; and obtaining the one or more wobble parameters based on the curve.

12. The system of clause 1, wherein: the diagnostic data includes an angle kappa factor.

13. The system of clause 1, wherein: the diagnostic data includes questionnaire data for the patient with at least one personality trait, the at least one personality trait being represented as at least one of a numerical scale of agreeability or as a binary result, the binary result being either predominantly agreeable or predominantly non-agreeable.

14. The system of clause 1, wherein: determining the respective satisfaction metric includes selectively executing at least one machine learning model trained with the respective historical sets; and the respective historical sets include pre-operative objective data, pre-operative personality data, intra-operative data, post-operative objective data, and subjective outcome data.

15. The system of clause 14, wherein: the subjective outcome data in the respective historical sets include a numerical satisfaction scale.

16. The system of clause 14, wherein: the controller is configured to quantify a correlation of the post-operative objective data to the subjective outcome score in the respective historical sets and identify the post-operative objective data most strongly correlating with the subjective outcome score.

17. A method of selecting a preferred intraocular lens for implantation in an eye, with a system having a controller with a processor and a tangible, non-transitory memory on which instructions are recorded, the method comprising: obtaining diagnostic data for the eye, via the controller; analyzing individual risk factors based on the diagnostic data, via the controller; obtaining historical data composed of respective historical sets of patient data; obtaining a weighted combination of the individual risk factors based in part on the historical data, via the controller; and generating a respective satisfaction metric for the plurality of intraocular lenses based on the historical data, via the controller.

18. The method of clause 17, further comprising: performing visual simulation for each of a plurality of intraocular lenses based in part on the diagnostic data, via the controller, the visual simulation including an impact of tear film data; and selecting the preferred intraocular lens based in part on the respective satisfaction metric and the weighted combination of the individual risk factors, via the controller.

19. The method of clause 17, further comprising: including lens capsule stability data in the diagnostic data, the lens capsule stability data being represented by one or more wobble parameters; and obtaining the lens capsule stability data by acquiring a plurality of images of the eye while presenting different accommodative demands to the eye and generating a motion trace of a lens capsule of the eye using the plurality of images.

20. A system for selecting a preferred intraocular lens for implantation into an eye, the system comprising: a controller having a processor and a tangible, non-transitory memory on which instructions are recorded, execution of the instructions causing the controller to:

obtain diagnostic data of the eye, including tear film data, the eye being scanned to generate the diagnostic data; perform a visual simulation for each of the plurality of intraocular lenses based in part on the diagnostic data, the visual simulation incorporating an impact of the tear film data; obtain historical data composed of respective historical sets of patient data; and generate a respective satisfaction metric for the plurality of intraocular lenses based in part on the visual simulation and the historical data.

**Claims**

1. A system for selecting a preferred intraocular lens, from a plurality of intraocular lenses, for implantation into an eye of a patient, the system comprising:
a controller having a processor and a tangible, non-transitory memory on which instructions are recorded, execution of the instructions causing the controller to:

obtain diagnostic data of the eye;
obtain historical data composed of respective historical sets of patient data;
analyze individual risk factors based on the diagnostic data and obtain a weighted combination of the individual risk factors based in part on the historical data; and
generate a respective satisfaction metric for the plurality of intraocular lenses based in part the historical data.

2. The system of claim 1, wherein the controller is configured to:
select the preferred intraocular lens based on the respective satisfaction metric and the weighted combination of the individual risk factors.

3. The system of claim 1, wherein the controller is configured to:

perform a visual simulation for each of the plurality of intraocular lenses based in part on the diagnostic data, the respective satisfaction metric being based in part on the visual simulation.

4. The system of claim 3, wherein:
the diagnostic data includes tear film data, the visual simulation incorporating an impact of the tear film data, including:

detecting a respective location where the tear film data exhibits at least one of a change in a signal-to-noise ratio and a relatively lower signal-to-noise ratio than that of surrounding locations; and
identifying the respective location as a respective irregularity of a tear film in the eye.

5. The system of claim 3, wherein:
the diagnostic data includes tear film data, the visual simulation incorporating an impact of the tear film data, including:

identifying a respective location where the tear film data exhibits at least one of missing information and a varying point distribution; and
identifying the respective location as a respective irregularity of a tear film.

6. The system of claim 1, wherein:

the diagnostic data include corneal data represented as at least one of a binary result or as a numerical scale of irregular corneal aberrations, the eye being scanned to generate the diagnostic data; and
the binary result is either a presence of a threshold level of corneal aberrations or an absence of the threshold level of corneal aberrations.

7. The system of claim 1, wherein:

the diagnostic data include macular data represented as at least one of a binary result or as a numerical scale of macular degeneration the eye being scanned to generate the diagnostic data; and
the binary result is either a presence of a threshold level of degeneration or an absence of the threshold level of degeneration.

8. The system of claim 1, wherein the diagnostic data include:

a respective location, orientation, and size of a pupil of the eye in a three-dimensional coordinate system, the pupil being under photopic conditions; and

the respective location and respective profile of an anterior corneal surface and a posterior corneal surface of the eye.

9. The system of claim 1, wherein:
the diagnostic data includes lens capsule stability data represented by one or more wobble parameters, obtaining the lens capsule stability data including:

acquiring a plurality of images of the eye while presenting different accommodative demands to the eye; and
generating a motion trace of a lens capsule of the eye using the plurality of images.

10. The system of claim 9, wherein obtaining the lens capsule stability data further includes:

extracting normalized lens oscillation traces based on the motion trace;
model-fitting a curve to the normalized lens oscillation traces; and
obtaining the one or more wobble parameters as a maximum amplitude and/or a time constant of the curve.

11. The system of claim 1, wherein: the diagnostic data includes at least one of the following:

(i) lens capsule stability data represented by one or more wobble parameters, obtaining the lens capsule stability data including:

directing electromagnetic energy in a predetermined spectrum onto the eye concurrently with induced eye saccades, via an energy source;
acquiring a plurality of images of the eye indicative of the induced eye saccades, via a camera;
generating a motion trace of a lens capsule using the plurality of images and extracting normalized lens oscillation traces based on the motion trace;
model-fitting a curve to the normalized lens oscillation traces; and
obtaining the one or more wobble parameters based on the curve.

(ii) the diagnostic data includes an angle kappa factor;
(iii) the diagnostic data includes questionnaire data for the patient with at least one personality trait, the at least one personality trait being represented as at least one of a numerical scale of agreeability or as a binary result, the binary result being either predominantly agreeable or predominantly non-agreeable.

12. The system of claim 1, wherein:

determining the respective satisfaction metric includes selectively executing at least one machine learning model trained with the respective historical sets; and
the respective historical sets include pre-operative objective data, pre-operative personality data, intra-operative data, post-operative objective data, and subjective outcome data, and optionally wherein
the subjective outcome data in the respective historical sets include a numerical satisfaction scale.

13. The system of claim 12, wherein:
the controller is configured to quantify a correlation of the post-operative objective data to the subjective outcome score in the respective historical sets and identify the post-operative objective data most strongly correlating with the subjective outcome score.

14. A system for selecting a preferred intraocular lens for implantation into an eye, the system comprising:
a controller having a processor and a tangible, non-transitory memory on which instructions are recorded, execution of the instructions causing the controller to:

the diagnostic data of the eye, including tear film data, the eye being scanned to generate the diagnostic data;
perform a visual simulation for each of the plurality of intraocular lenses based in part on the diagnostic data, the visual simulation incorporating an impact of the tear film data;
obtain historical data composed of respective historical sets of patient data; and
generate a respective satisfaction metric for the plurality of intraocular lenses based in part on the visual simulation and the historical data.

15. The system of claim 14, further comprising:

detecting a respective location where the tear film data exhibits at least one of a change in a signal-to-noise ratio and a relatively lower signal-to-noise ratio than that of surrounding locations; and
identifying the respective location as a respective irregularity of a tear film in the eye;

FIG. 1

100

S

102 — Obtain diagnostic data

104 — Select IOL models

106 — Perform vision simulation

108 — Obtain historical data

110 — Analyze risk factors

112 — Obtain weighted combination of risk factors

114 —

116 — Obtain respective satisfaction metric

118 — Perform risk analysis

E

**FIG. 2**

**FIG. 3**

**FIG. 4**

FIG. 5

EP 4 738 378 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 63126441 A

- WO 63129386 A

**Non-patent literature cited in the description**

- **MCCRAE, R.** ; **COSTA, P**. Personality in Adulthood: A Five-Factor Theory Perspective. Guilford Press, 2003